# EUROPEAN PATENT APPLICATION

(11) **EP 4 265 241 A1**
(43) Date of publication of application: **25.10.2023**
(21) Application number: 22169120.7
(22) Date of filing: 20.04.2022
(51) Int. Cl.: A61K 9/20, A61K 31/55, A61P 13/02

(54) **TABLET FORMULATIONS FOR THE TREATMENT OF URINARY SYSTEM INFECTIONS AND METHOD FOR THE PREPARATION OF SUCH FORMULATIONS**

(71) Applicant: Bilim Ilac Sanayii Ve Ticaret A.S., 34440 Istanbul (TR)
(72) Inventor: Safadi, Muhammad, Istanbul (TR); Duran Ozel, Arzu, 34440 Istanbul (TR)
(74) Representative: Mutlu, Aydin

(57) **Abstract**

The present invention relates to bisect tablet formulations comprising pivmecillinam and a preparation method thereof.

## Description

### Technical Field

The present invention relates to a bisect tablet formulation comprising a beta-lactam antibiotic for use in the treatment of urinary tract infections. More particularly, the present invention provides a bisect tablet formulation comprising pivmecillinam for use in the treatment of urinary tract infections and a preparation method thereof.

### Background of the Invention

Pivmecillinam or amdinocillin pivoxil is an ester of mecillinam [(2,2-dimethylpropanoyl)oxy]methyl ester and prodrug of penicillanic acid. The chemical structure of pivmecillinam, which is used as an anti-infective agent and antibacterial drug, is given in Formula I below.

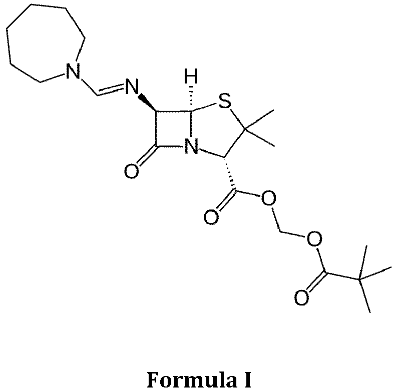

Pivmecillinam is considered active only against Gram-negative bacteria and is also used in the treatment of lower urinary tract infections. This indication has been widely used in Scandinavian countries since the 1970s. It is recommended as the first choice for the empirical treatment of acute cystitis. It is also used in the treatment of paratyphoid fever and shigellosis. The drug with the active substance in the market is available under the trade names Selexid^{®}, Penomax^{®} and Coactabs^{®}.

Empirical treatment can be started directly for the treatment of acute cystitis and pyelonephritis, which are clinically symptomatic but without signs of vaginal discharge and irritation. For this purpose, pivmecillinam is recommended as an empirical antibiotic for the treatment of uncomplicated urinary tract infections as a first choice.

US 3957764 A relates to pivmecillinam and amidino penicillin derivatives. The document discloses FL1039 (pivmecilllinam) tablet formulations. US 3755588 A relates to formulations of pivmecillinam for tablets and injections in the dose range of 0.025 g to 1 g. US 5958453 A relates to a solid pharmaceutical preparation which exhibits a fast buccal disintegratability and dissolubility in the oral cavity especially even without water. The document discloses formulations comprising the active ingredient, erythritol, crystalline cellulose and crospovidone. TR 1998/01824 relates to β-lactam antibiotic agglomerates without excipients.

Although pivmecillinam tablet formulations are available in the state of the art, it is not possible to use them with appropriate dose titrations since the dose requirement differs from patient to patient. According to the dose opinion of physicians, it is not possible to use standard tablets with flexible doses. As a result, undesirable side effects or, on the contrary, undesirable results such as low bioavailability are encountered. The present invention aims to present bisect tablets of pivmecillinam based on these long-reported complaints by physicians. Thus, it is possible to split the tablets by breaking and use them in the optimum dose as prescribed.

Another problem experienced in pivmecillinam tablet formulations in the state of the art is that the formulations do not have sufficient homogeneous dispersibility, high solubility and stability. Although the known tablet formulations supply precautions to overcome the stability problem, incompatibility problems with the excipients in the formulations are ongoing. In addition, sufficient resistance against external factors such as heat, light and humidity cannot be provided. Therefore, there is a continous need for pivmecillinam tablet formulations with high stability, durability and dissolution rate in the art

In the light of the above information, it can be seen that there is a need in the related technical field for pharmaceutical compositions with high solubility as well as preparation methods thereof, which provide high stability and durability of compositions comprising pivmecillinam. For this purpose, within the scope of the present invention, a bisect tablet formulation comprising pivmecillinam for use in the treatment of urinary tract infections and a preparation method thereof are provided.

### Summary of the Invention

In an aspect, the present invention provides a bisect tablet formulation comprising pivmecillinam for use in the treatment of urinary tract infections. Further, the formulation of the present invention preferably comprises at least one pharmaceutically acceptable excipient

The at least one pharmaceutically acceptable excipient according to the present invention is preferably selected from the group consisting of a superdisintegrant, gelling agent, tablet diluent, carrier, and combinations thereof. Said superdisintegrant is preferably selected from the group consisting of polyplasdone XL, polyplasdone XL 10, kollidon CL, croscarmellose sodium and sodium starch glycolate. Said gelling agent is preferably selected from the group consisting of magnesium stearate, sodium arginate, guar gum, gum arabic, xanthan gum, sodium carboxymethyl cellulose, carbomer, carboxymethylcellulose sodium, dextrin, dextrose, maltodextrin, gelatin, copovidone, alginic acid and carrageenan. The tablet diluent of the present invention is preferably selected from the group consisting of microcrystalline cellulose, pregelatinized starch, starch, carboxymethylcellulose calcium, magnesium aluminum silicate, methylcellulose, hydroxypropyl cellulose, carboxymethylcellulose sodium, crospovidone, low substituted hydroxypropyl cellulose, sodium alginate, and silicified microcrystalline cellulose. Said carrier is preferably selected from the group consisting of meglumine, ethylene glycol, glycerol, erythritol, threitol, arabitol, xylitol, ribitol, mannitol, lactose, sorbitol, galacticol, glucose anhydrate, cellobiose and maltitol.

The amount of pivmecillinam in the formulation of the present invention is preferably in the range of 200-1000 mg. More preferably, the amount of pivmecillinam is about 400 mg.

In another aspect, the present invention provides a method for producing a bisect tablet formulation comprising pivmecillinam, the method comprises the following steps:
- stirring a mixture comprising pivmecillinam and at least one pharmaceutically acceptable excipient with a solvent,
- granulating of the obtained wet mixture,
- sieving and drying of the obtained granules,
- compressing of the dry composition as bisect tablets, and
- obtaining a bisect tablet.

### Detailed Description of the Invention

In this detailed description, the bisect tablet formulation comprising the beta-lactam antibiotic according to the present invention is described for a better understanding of the subject. In an aspect, the present invention provides a bisect tablet formulation for use in the treatment of urinary tract infections, the formulation comprising a beta-lactam antibiotic. Within the scope of the invention, with the tablet formulation designed with pharmaceutically acceptable excipients, a solution is presented to the problems that occur in situations such as dissolution rate, compressibility, stability, durability, and a tablet formulation with higher dissolution rate, compressibility, stability and durability compared to the formulations in the related technical field is provided.

The pharmaceutical composition of the present invention comprises therapeutic amounts of beta-lactam antibiotics. The beta-lactam antibiotic is preferably selected from the broad spectrum penicillin group. The broad spectrum penicillin group antibiotic is preferably selected from among pivmecillinam, amoxicillin, ampicillin, pivampicillin, ticarcillin and temosillin. More preferably, said broad spectrum penicillin is pivmecillinam.

In preferred embodiments of the present invention, the active ingredient of pivmecillinam is preferably in the range of 200-1000 mg. The amount of pivmecillinam can be 200, 300, 400, 500, 600, 700, 800, 900 or 1000 mg. More preferably, the amount of pivmecillinam is about 400 mg. The present inventors report that the use of the relevant active ingredient in this amount makes the tablet formulation suitable for use according to the invention, and it becomes more effective in the treatment of urinary tract infections.

Preferred embodiments of the present invention can comprise at least one pharmaceutically acceptable excipient Said pharmaceutically acceptable excipient can preferably be selected from the group consisting of superdisintegrant, gelling agent, tablet diluent, carrier, or combinations thereof.

In the present formulation, superdisintegrants are advantageous in that they can be used in smaller amounts compared to conventional disintegrants. Therefore, the use of lesser amounts of superdisintegrants benefits the dissolution rate of the formulation over conventional disintegrants. Therefore, in preferred embodiments of the present invention, it is aimed to increase the dissolution rate of the formulation by adding superdisintegrants to the formulation. Within the scope of the present formulation, a desirable effect is revealed as a result of combining pivmecillinam with a superdisintegrant to form a tablet formulation. Herein said superdisintegrant can preferably be selected from the group consisting of polyplasdone XL, polyplasdone XL 10, kollidon CL, croscarmellose sodium and sodium starch glycolate.

Gelling agents are also known in the state of art as thickeners or viscosity adjusters. In preferred embodiments of the present invention, the formulation can comprise a gelling agent Said gelling agent can preferably be selected from the group consisting of magnesium stearate, sodium arginate, guar gum, gum arabic, xanthan gum, sodium carboxymethyl cellulose, carbomer, carboxymethylcellulose sodium, dextrin, dextrose, maltodextrin, gelatin, copovidone, alginic acid and carrageenan.

The formulation of the present invention can comprise at least one pharmaceutically acceptable tablet diluent. The tablet diluent of the present invention can be selected from the group consisting of microcrystalline cellulose, pregelatinized starch, starch, carboxymethylcellulose calcium, magnesium aluminum silicate, methylcellulose, hydroxypropyl cellulose, carboxymethylcellulose sodium, crospovidone, low substituted hydroxypropyl cellulose, sodium alginate, and silicified microcrystalline cellulose.

The formulation of the present invention can comprise at least one pharmaceutically acceptable carrier. Said pharmaceutically acceptable carrier is preferably selected from the group consisting of meglumine, ethylene glycol, glycerol, erythritol, threitol, arabitol, xylitol, ribitol, mannitol, lactose, sorbitol, galacticol, glucose anhydrate, cellobiose, maltitol, and mixtures thereof and also it is not limited to only these sugar alcohols or sugar alcohol derivatives. More preferably said carrier can be meglumine.

Meglumine is a sugar alcohol derived from glucose containing an amino group modification. It is often used with iodinated compounds as an excipient in pharmaceuticals and in contrast agents such as diatrizoate meglumine, iothalamate meglumine, and iodipamide meglumine.

In another aspect, the present invention provides a method for producing a bisect tablet formulation, the method comprises the following steps:
- stirring a mixture comprising pivmecillinam and at least one pharmaceutically acceptable excipient with a solvent,
- granulating of the obtained wet mixture,
- sieving and drying of the obtained granules,
- compressing of the dry composition as bisect tablets, and
- obtaining a bisect tablet.

The solvent in the method of the present invention can preferably be selected from the group consisting of ethanol, isopropyl alcohol, water or a combination thereof.

The wet mixture in the method is granulated by spraying method. In the method of the present invention, in the said step of "sieving and drying the obtained granules", the granules can be sieved with a 40 mesh sieve, and also the wet granules can be dried at a temperature in the range of 45-60°C.

Herein, the amount of pivmecillinam is in the range of 70-80% by weight of the overall tablet weight. Also, the amount of superdisintegrant of the present invention is in the range of 6-12% by weight, the amount of gelling agent is 1-3% by weight, the amount of tablet diluent is in the range of 6-12% by weight, and the amount of carrier is in the range of 2-4% by weight of the total tablet formulation.

Further embodiments of the present invention can also comprise pharmaceutically acceptable excipients, solvents, binders, lubricants, absorbants, fillers, surfactants, flavoring agents, aromatics, and film coaters which are suitable for use as provided according to the invention apart from those mentioned above.

Therefore, the present invention is advantageous because it presents a tablet formulation with good dispersion and stability, and in this aspect it provides an improvement over the state of art. The desirable effect of the pharmaceutical composition of the present invention benefits the rate of solubility and is therefore more advantageous compared to existing methods.

In another preferred embodiment of the present invention, a desirable effect occurs as a result of combining the active ingredient of pivmecillinam and the superdisintegrant. Thus, the tablet formulation of the invention can be obtained with suitable hardness. In addition, with the wet granulation method, the segregation of the components during the tableting process is also prevented. Therefore, a composition with improved solubility, high stability and durability is provided by the present invention.

In another aspect of the invention, a bisect tablet formulation of the present invention is provided for use in the treatment of urinary tract infections.

The following findings were reached as a result of clinical studies of pivmecillinam available in the prior art.

One hundred and eighty-four women, with acute uncomplicated cystitis, received a 3-day course of pivmecillinam comprising an initial 400 mg (two tablets) dose, followed by 200 mg every 8 hours; a total of ten tablets. A satisfactory clinical response was achieved in 91% of patients. Bacteriological success was observed in 94% of sixty-eight patients with a proven infection. Side-effects were reported in sixteen patients (8.4%) and two patients ceased therapy at an early stage (J.,F., Donald, 1980, J. Int. Med. Res., 8, 112*).*

Pivmecillinam (FL 1039) is the pivaloyloxymethyl ester of mecillinam (FL 1060) which has considerable in vitro activity against *Enterobacteriaceae.* 38 hospital inpatients who had proven urinary tract infections were treated with 400 mg pivmecillinam four times daily for 5-7 days. The MIC of mecillinam to the infecting organisms was determined as were the serum and urinary concentrations of the antibiotic. The patients were followed up for 4-6 weeks after the end of treatment. Three patients were ceased to be followed-up. Of the 35 patients who were adequately followed up, 29 (83%) were classified as cured and there were 6 failures. Reported side effects were of a minor nature *(*R. Wise, 1976, Chemotherapy, 22:335-339*).*

### EXAMPLES

Bisect tablet formulations comprising pivmecillinam were prepared by following the procedure below:
A therapeutic amount of pivmecillinam and pharmaceutically acceptable excipients that are weighed and mixed in selected amounts are added to microcrystalline cellulose, hypromellose and simethicone together with water to ensure a homogeneous consistency of the mixture. The granulation process of the wet mixture obtained is performed. The granules are first sieved through a 40 mesh sieve and then dried at 45 °C. Magnesium stearate, which is weighed in an appropriate amount, is sieved through a 60 mesh sieve and added on the obtained granules and mixed for 3 minutes at an appropriate speed. Tablet compression of the dry composition obtained is performed.

In preferred embodiments, the pharmaceutical composition of the present invention comprises the following components:

| **Component** | **Percentage by weight (%)** |
|---|---|
| Pivmecillinam | 70-80 |
| Superdisintegrant | 6-12 |
| Tablet diluent | 6-12 |
| Gelling agent | 1-3 |
| Carrier | 2-4 |

The pharmaceutical composition of the present invention preferably comprises the following components:

| **Component** | **Amount (mg)** |
|---|---|
| Pivmecillinam | 200-400 |
| Superdisintegrant | 10-60 |
| Tablet diluent | 10-60 |
| Gelling agent | 2-10 |
| Carrier | 10-40 |

### Example 1 - Pivmecillinam 200 mg Tablet Formulation

| **Component** | **Amount (mg)** | **Percentage by weight (%)** |
|---|---|---|
| Pivmecillinam | 200.00 | 76.5 |
| Microcrystalline cellulose | 22.90 | 8.76 |
| Hypromellose | 22.90 | 8.76 |
| Magnesium stearate | 3.50 | 1.33 |
| Simethicone | 12.00 | 4.6 |
| Synthetic paraffin | q.s. | |
| **Total** | 261.30 | 100 |

### Example 2 - Pivmecillinam 200 mg Tablet Formulation

| **Component** | **Amount (mg)** | **Percentage by weight (%)** |
|---|---|---|
| Pivmecillinam | 200.00 | 73.8 |
| Hydroxypropyl cellulose | 28.60 | 10.6 |
| Polyplasdone XL | 27.80 | 10.3 |
| Calcium stearate | 4.30 | 1.6 |
| Copovidone | 10.00 | 3.7 |
| Synthetic paraffin | q.s. | |
| **Total** | 270.7 | 100 |

### Example 3 - Pivmecillinam 400 mg Tablet Formulation

| **Component** | **Amount (mg)** | **Percentage by weight (%)** |
|---|---|---|
| Pivmecillinam | 400.00 | 77.7 |
| Microcrystalline cellulose | 42.90 | 8.3 |
| Hypromellose | 42.90 | 8.3 |
| Magnesium stearate | 6.50 | 1.3 |
| Simethicone | 22.00 | 4.27 |
| Synthetic paraffin | q.s. | |
| **Total** | 514.30 | 100 |

### Example 4 - Pivmecillinam 400 mg Tablet Formulation

| **Component** | **Amount (mg)** | **Percentage by weight (%)** |
|---|---|---|
| Pivampicillin | 400.00 | 77.2 |
| Hydroxypropyl cellulose | 46.00 | 8.8 |
| Polyplasdone XL | 44.00 | 8.5 |
| Calcium stearate | 8.50 | 1.6 |
| Copovidone | 19.00 | 3.7 |
| Synthetic paraffin | q.s. | |
| **Total** | 517.5 | 100 |

The present invention is advantageous in that it provides tablet formulations with good dispersibility, high solubility and stability, beside sufficient resistance to external factors such as heat, light and moisture, with the specific composition of the active ingredient of pivmecillinam combined with pharmaceutically acceptable excipients.

The scope of protection of the invention is specified in the appended claims, and it is clear that a person skilled in the art can put forward similar embodiments in the light of the above, without deviating the scope of the claims.

## Claims

1. A bisect tablet formulation of pivmecillinam for use in the treatment of urinary tract infections.

2. The formulation according to claim 1, **characterized in that** the bisect tablet formulation comprises at least one pharmaceutically acceptable excipient which is selected from the group consisting of a superdisintegrant, gelling agent, tablet diluent, carrier or combinations thereof.

3. The formulation according to claim 2, **characterized in that** the superdisintegrant is selected from a group consisting of polyplasdone XL, polyplasdone XL 10, kollidon CL, croscarmellose sodium and sodium starch glycolate.

4. The formulation according to claim 2, **characterized in that** the gelling agent is selected from the group consisting of magnesium stearate, sodium arginate, guar gum, gum arabic, xanthan gum, sodium carboxymethyl cellulose, carbomer, carboxymethylcellulose sodium, dextrin, dextrose, maltodextrin, gelatin, copovidone, alginic acid and carrageenan.

5. The formulation according to claim 2, **characterized in that** the tablet diluent is selected from the group consisting of microcrystalline cellulose, pregelatinized starch, starch, carboxymethylcellulose calcium, magnesium aluminum silicate, methylcellulose, hydroxypropyl cellulose, carboxymethylcellulose sodium, crospovidone, low substituted hydroxypropyl cellulose, sodium alginate and silicified microcrystalline microcrystalline cellulose.

6. The formulation according to claim 2, **characterized in that** the carrier is selected from a group consisting of meglumine, ethylene glycol, glycerol, erythritol, threitol, arabitol, xylitol, ribitol, mannitol, lactose, sorbitol, galacticol, glucose anhydrate, cellobiose and maltitol.

7. The formulation according to claim 1, **characterized in that** the formulation comprises the following components:
| **Component** | **Percentage by weight (%)** |
|---|---|
| Pivmecillinam | 70-80 |
| Superdisintegrant | 6-12 |
| Tablet diluent | 6-12 |
| Gelling agent | 1-3 |
| Carrier | 2-4 |

8. The formulation according to claim 7, **characterized in that** the amount of pivmecillinam of the formulation is in the range of 200-1000 mg.

9. The formulation according to claim 8, **characterized in that** the formulation comprises the following components:
| **Component** | **Amount (mg)** |
|---|---|
| Pivmecillinam | 200-400 |
| Superdisintegrant | 10-60 |
| Tablet diluent | 10-60 |
| Gelling agent | 2-10 |
| Carrier | 10-40 |

10. A method for preparing a bisect tablet formulation comprising pivmecillinam, **characterized in that** the method comprises the following steps:
- stirring a mixture comprising pivmecillinam and at least one pharmaceutically acceptable excipient with a solvent,
- granulating of the obtained wet mixture,
- sieving and drying of the obtained granules,
- compressing of the dry composition as bisect tablets, and
- obtaining a bisect tablet.

11. The method according to claim 10, **characterized in that** the wet mixture of the method is granulated by spraying method.

12. The method according to claim 10, **characterized in that** the obtained granules of the method are sieved with a 40 mesh sieve.

13. The method according to claim 10, **characterized in that** the wet granules of the method are dried at a temperature between 45-60°C.

14. The method according to claim 10, **characterized in that** the solvent of the method is selected from a group consisting of ethanol, isopropyl alcohol, water and combinations thereof.
